# FASCICULE DE BREVET EUROPEEN

(11) **EP 2 109 438 B1**
(45) Date de publication et mention de la délivrance du brevet: **03.11.2010**
(21) Numéro de dépôt: 07871883.0
(22) Date de dépôt: 06.12.2007
(51) Int. Cl.: A61K 8/19, A61K 8/25, A61Q 1/02

(54) **COMPOSITION DE MAQUILLAGE DE LA PEAU**
KOSMETISCHE ZUSAMMENSETZUNG ZUM HAUTSCHMINKEN
MAKE-UP COMPOSITION FOR THE SKIN

(30) Priorité: 07.12.2006 FR 0655376; 07.12.2006 US 873266 P
(43) Date de publication de la demande: 21.10.2009
(73) Titulaire: L'Oréal, 75008 Paris (FR)
(72) Inventeur: Teboul, Karen, F-94160 St Mande (FR); Cassin , Guillaume, F-91140 Villebon sur Yvette (FR)
(74) Mandataire: Leonard, Armelle
(86) Numéro de dépôt international: PCT/FR2007/052449
(87) Numéro de publication internationale: WO 2008/096085

(56) Documents cités:
- EP-A- 1 433 460
- DATABASE BIOSIS [Online] BIOSCIENCES INFORMATION SERVICE, PHILADELPHIA, PA, US; octobre 2001 (2001-10), ALATRACHE A ET AL: "[Ritual red cosmetic pigments based on cinnabar and ochre from the Punic era in Tunisia: Analysis, identification and characterization]" XP002445779 Database accession no. PREV200100541789 & INTERNATIONAL JOURNAL OF COSMETIC SCIENCE, vol. 23, no. 5, octobre 2001 (2001-10), pages 281-297, ISSN: 0142-5463
- PARISH L C ET AL: "Cosmetics: A historical review" CLINICS IN DERMATOLOGY, J.B. LIPPINCOTT, PHILADELPHIA, PA, US, vol. 6, no. 3, 1 juillet 1988 (1988-07-01), pages 1-4, XP023113087 ISSN: 0738-081X [extrait le 1988-07-01]
- DATABASE WPI Week 199944 Thomson Scientific, London, GB; AN 1999-522578 XP002445784 & JP 11 228349 A (NOEVIR KK) 24 août 1999 (1999-08-24)
- DATABASE WPI Week 200635 Thomson Scientific, London, GB; AN 2006-338304 XP002445785 & JP 2006 124335 A (PAK J Y) 18 mai 2006 (2006-05-18)

## Description

La présente invention a pour objet une composition cosmétique comprenant au moins une terre colorante et une charge matifiante. L'invention a également pour objet un procédé de maquillage ou de soin des matières kératiniques, et notamment de la peau, ou des lèvres comprenant l'application de la composition sur les matières kératiniques.

La composition selon l'invention peut être une composition de maquillage et/ou de soin des matières kératiniques, en particulier une composition de soin ou de maquillage de la peau, telle qu'une crème de jour, un fond de teint, un fard à paupières, un fard à joue, un produit anticernes, une poudre du visage et du corps, un produit de maquillage du corps.

Les compositions de maquillage ou de soin comprennent généralement, d'une part, une phase pulvérulente comportant notamment des charges et éventuellement des pigments et d'autre part, une phase grasse comprenant des corps gras, destinée à conférer au produit fini une certaine densité, à donner une douceur et une propriété émolliente au produit et à favoriser son adhérence sur la peau.

Après l'application d'une crème ou d'un maquillage sur la peau, le sébum excrété par la peau au cours du temps modifie les propriétés cosmétiques du maquillage. En particulier, le sébum ne favorise pas l'adhésion du maquillage sur la peau rendant le dépôt de maquillage non homogène. En outre, le dépôt devient également plus brillant ; ce qui n'est pas recherché pour des compositions de maquillage ou de soin de la peau, notamment du visage, les consommatrices préférant un maquillage mat et conservant cette matité pendant la journée.

Il est ainsi connu d'utiliser certaines charges pour obtenir un effet de matité. Ces charges sont le plus souvent choisies en fonction de leurs bonnes propriétés d'absorption des huiles et/ou de leur capacité à diffuser la lumière de manière efficace. Cependant, l'effet de matité est souvent altéré voire totalement masqué par les autres ingrédients de la formule et notamment par les pigments ou des charges opaques.

Il existe donc un besoin pour des compositions cosmétiques de maquillage ou de soin des matières kératiniques et notamment de la peau qui conférant un effet matifiant lorsqu'elles appliquées sur lesdites matières kératiniques.

Les inventeurs ont mis en évidence qu'il était possible de formuler une telle composition cosmétique c'est-à-dire dotée d'un obtenir un effet matifiant, en associant des charges matifiantes avec des terres colorantes.

L'invention a donc pour objet, selon un premier aspect, une composition cosmétique comprenant, dans un milieu physiologiquement acceptable, au moins une terre colorante et au moins une charge matifiante.

L'invention a également pour objet, selon un deuxième aspect, un procédé de maquillage ou de soin des matières kératiniques, et notamment de la peau, ou des lèvres comprenant l'application de la composition sur les matières kératiniques.

Enfin, l'invention a pour objet, selon un troisième aspect, l'utilisation d'une composition telle que décrite précédemment pour obtenir un dépôt mat.

### Terres colorantes

La composition selon l'invention comprend au moins une terre colorante.

### 1. Définition générale

Par « terres colorantes » au sens de la présente demande, on entend des particules composites, existant à l'état naturel, comprenant une partie minérale colorante (pigments naturels) et une partie minérale non colorante (charges naturelles).

Ces terres colorantes peuvent, en vue d'être incorporées dans des compositions cosmétiques selon l'invention, faire l'objet d'un certain nombre de transformations, notamment après extraction, qui seront détaillées par la suite.

Dans la présente demande, on emploie indifféremment le terme minéral ou inorganique.

Les terres colorantes utilisées dans le cadre de la présente invention peuvent être des composés minéraux naturels pulvérulents constitués de « charges » naturelles dans lesquelles sont dispersés des « pigments », lesquels pigments peuvent être naturels ou d'origine naturelle.

Ces terres colorantes sont de préférence d'origine naturelle. Toutefois, elles peuvent être fabriquées industriellement, l'essentiel étant dans ce dernier cas qu'elles présentent une structure identique ou similaire à des structures composites d'origine naturelle (c'est-à-dire comprenant une partie minérale colorante et une partie minérale non colorante) et que, de ce fait, elles présentent des propriétés physico-chimiques identiques ou similaires aux terres colorantes.

De préférence, le terme « terres colorantes» n'englobe pas les particules qui sont couvertes ou enrobées par une couche ou plusieurs couches de pigments sur leur surface.

On entend par « charge naturelle » au sens de la présente demande, des particules minérales non colorantes.

On entend par « pigment naturel » au sens de la présente demande, des particules minérales colorantes.

Par « partie minérale colorante», on entend des particules colorées dans la masse « aptes à conférer une couleur » autre que le blanc à la composition dans laquelle elles se trouvent. La composition comprenant des « particules minérales colorantes» est apte à colorer le support sur lequel elle est appliquée. Ainsi, la composition obtenue colorante, et de manière préférentielle, colorée. On entend donc par particules colorantes des particules aptes à colorer le support sur lequel elles sont appliquées.

Les particules minérales colorantes présentent une couleur autre que le blanc. Elles sont insolubles dans les autres ingrédients de la composition cosmétique, destinées à colorer et opacifier le film de composition cosmétique déposé sur les matières kératiniques.

Par « partie minérale non colorante » ou « charges naturelles », on entend des particules incolores ou blanches, de forme lamellaire ou non lamellaire. Ces particules ne confèrent pas de couleur (autre que le blanc) à la composition dans laquelle elles se trouvent. Ainsi, la composition comprenant uniquement des particules non colorantes n'est ni colorée, ni colorante. On entend donc par particules « non colorantes » des particules ne colorant pas le support sur lequel elles sont appliquées dans une couleur autre que le blanc.

### 2. Pigments et charges d'origine naturelle

Les terres colorantes peuvent avoir fait l'objet de transformations, ces transformations pouvant être :
1) des transformations ne modifiant pas la composition de la matière première par rapport à son origine hormis éventuellement sa teneur en eau. Ces transformations engendrent essentiellement des modifications de l'aspect physique de l'ingrédient par rapport à son origine. Des exemples de transformations entrant dans cette catégorie sont :
   - le lavage
   - le concassage,
   - le broyage,
   - le séchage,
   - la lyophilisation,
   - les procédés thermiques de conservation (appertisation, - pasteurisation),
   - les procédés de conservation par pression (Pascalisation),
   - l'addition de conservateurs d'origine végétale étant acceptée.
2) Dans le cas des matières minérales, les procédés de transformation peuvent être les suivants :
   - les procédés visant à purifier ou modifier légèrement la matière première sans modification significative de sa structure cristalline ou de sa composition,
   - la distillation,
   - les procédés de purification (élimination de métaux lourds, de composés organiques...),
   - les procédés d'échanges ioniques,
   - les procédés de purification par passage sur charbon actif, sur oxydes ou sur résine,
   - les procédés thermiques de conservation,
   - les procédés de conservation par pression (Pascalisation),
3) pour les ingrédients d'origine minérale : les procédés permettant d'obtenir des matériaux par dissolution re-précipitation d'espèces minérales conduisant à des oxydes simples ou structurés (zéolites, mésoporeux...),
4) des transformations pour une pour une fonctionnalisation, notamment l'amination, la nitration, la sililation, la carboxylation en utilisant des catalyseurs d'origine minérale ou biologique, ainsi que les bio transformations par des organismes génétiquement modifiés dont la fonction correspond ou non à la réaction originelle, et les procédés donnant lieu à la synthèse de mélanges d'oxydes.

### 3. Classement des terres colorantes

Les terres colorantes pouvant convenir à la présente invention peuvent être classées en fonction de leur couleur, en fonction de leur provenance géographique ou en fonction de leur composition chimique.

Les terres colorantes selon l'invention sont, de préférence, des particules composites dont la partie minérale colorante comprend du fer, de préférence sous forme oxydée.

En particulier, les terres colorantes peuvent comprendre entre 3% et 95% en poids d'oxyde de fer exprimé en oxyde ferrique Fe₂O₃, de préférence entre 5 et 80 %, et plus préférentiellement entre 5 et 65%.

Dans la littérature, le terme ocre est parfois utilisé pour désigner certaines terres colorantes. En principe, le terme « ocre » devrait désigner une sous catégorie de terres colorantes comprenant une teneur en oxyde de fer assez faible. Dans la pratique, le terme « ocre » est utilisé au même titre que le terme « terre colorante » sans que soit précisée la teneur en oxyde de fer.

Les différents oxydes de fer naturels sont notamment identifiés selon la norme NFT 36.001 de 1988. On distingue notamment :
- les oxydes de fer jaunes naturels (Pigment Yellow 43) identifiés par les références du Colour Index C.I 77492 : il s'agit d'oxydes de fer hydratés (goethite, lépidocrocite),
- les oxydes de fer rouges naturels (Pigment Red 102) identifiés par les références du Colour Index C.I 77491 : il s'agit de sesquioxyde de fer naturel du type Hématite, et
- les oxydes de fer noirs naturels (Pigment Black 11) identifiés par les références du Colour Index C.I 77499 : il s'agit de magnétite chargée en phosphore et soufre.

Les oxydes de fer les plus présents dans les terres peuvent notamment être sous forme hydratée appelée Goethite (Oxyde de fer jaune) ou sous forme anhydre appelée Hématite (Oxyde de fer rouge). L'Hématite existe à l'état natif, et elle peut être aussi obtenue après calcination de la Goethite. Il est ainsi possible, en fonction des conditions opératoires du procédé de calcination, de faire varier le ratio Goethite/Hématite pour obtenir une gamme de couleurs de la terre colorante variant du jaune clair au rouge.

Les terres colorantes peuvent également comprendre des oxydes métalliques colorés autres que l'oxyde de fer, tels que l'oxyde de manganèse ou l'oxyde d'aluminium.

Les terres colorantes selon l'invention sont, de préférence, des particules composites dont la partie minérale non colorante peut être choisie parmi l'argile, du gypse, la calcite, le carbonate de calcium ou de magnésium, le quartz, l'alumine hydratée, la silice, la kaolinite et leurs mélanges.

Comme décrit précédemment, les terres colorantes selon la présente invention peuvent être classées en fonction de leur couleur. On peut distinguer les terres jaunes, les terres rouges, les terres vertes, les terres brunes ou les terres noires.

### Les terres jaunes

Le principal ingrédient des terres jaunes est l'oxyde de fer jaune. Parmi les terres jaunes présente à l'état natif, on peut citer :
- les terres jaunes provenant d'Italie, comme la terre de Sienne naturelle,
- les terres jaunes provenant de France, comme les grés du type "ocre de Puisaye",
- les terres jaunes provenant d'Amérique,
- l'ocre du Royaume Uni (jaune anglais) ou l'ocre d'Oxford, n'est plus extrait depuis longtemps.
- les terres ocres jaunes d'Espagne.

Les variétés les plus réputées d'ocre jaune provenaient de l'Yonne et d'autres régions de la moitié nord de la France mais aussi du sud de la France (Vaucluse).

Par exemple l'ocre jaune du Vaucluse est constituée de quartz, de kaolinite (argile) et d'oxyde de fer jaune naturel

L'ocre jaune naturelle peut être lavée (procédé de lévigation).

L'ocre est très souvent prélevée dans des sols sableux. Il faut donc séparer l'ocre du sable. La matière délayée est ensuite récupérée dans des bassins de décantation, puis séchée ou traitée, comme indiqué ci-dessus, par lévigation, et broyée.

### Les terres rougets

A titre d'exemple de terres colorantes rouges, on peut citer de manière non limitative l'ocre rouge du Vaucluse, le rouge vénitien, le rouge indien, et le rouge anglais.

Selon la littérature, le rouge vénitien, le rouge indien et le rouge anglais, sont tous des rouges opaques très colorants. L'intensité de leur rouge est bien plus importante que celle du rouge du Vaucluse. Le rouge anglais est un oxyde de fer rouge adjoint d'aluminosilicate de sodium polysulfuré, d'où son aspect assez violacé. Le rouge indien est très proche du rouge anglais, à tel point qu'il est difficile de les distinguer selon les différentes fabrications. Certaines variétés sont couvrantes et colorantes.

D'autres exemples de terres colorantes rouges sont la terre de Sienne brûlée, le rouge de Pouzzoles (terra rosa de Pozzuoli, ville proche de Naples), le rouge d'Ercolano (Italie), le rouge de Falun (ville de Suède dotée d'un gisement), le bol d'Arménie (terre colorante rouge aussi qualifiée d'ocre rouge).

### Les terres colorantes vertes

Les terres vertes comprennent des silicates complexes comme :
- la Glauconite : silicate complexe d'aluminium et de potassium contenant du fer, qui substitue plus ou moins l'aluminium, et
- la Céladonite : silicate d'aluminium, de magnésium et de sodium contenant du fer.

Les terres colorantes vertes peuvent également contenir du cuivre.

### Les terres brunes ou terres d'ombre

A l'état non calciné, les terres d'ombre présentent une couleur bleue-verte. Leurs variétés calcinées donnent un marron foncé proche d'un marron chocolat. La calcination provoque une déshydratation similaire à celle qui se produit lorsque l'on cuit une terre jaune pour obtenir une terre de couleur rouge.

La terre d'ombre de Cologne et la terre d'ombre de Chypre sont les plus vertes. Plusieurs autres variétés proviennent du Vaucluse (France). Il existe aussi des gisements en Ombrie, province d'Italie située au Sud de la Toscane et au Nord du Latium.

Les terres colorantes utilisées dans les compositions cosmétiques de l'invention sont notamment décrites dans l'ouvrage intitulé « Les cahiers de Terres et Couleurs » (réédition n°3 Février 1998) réalisé par le Centre de Recherche et de Restauration des Musées de France.

Les terres colorantes décrites précédemment comprennent également au moins partie minérale non colorante ou charge comme par exemple de l'argile (kaolinite), du gypse (sulfate de calcium), de la calcite (carbonate de calcium), du carbonate de magnésium, du quartz (silice cristalline), de-l'alumine hydratée, ou un de leurs mélanges.

En particulier, les terres colorantes convenant particulièrement à la présente invention comprennent au moins une partie minérale colorante et au moins une partie minérale non colorante, la partie minérale colorante étant solidaires avec l'un au moins des constituants de la partie minérale non colorant. De préférence, la partie minérale colorante est dispersée de façon homogène à l'intérieur de la matrice constituée par la partie minérale non colorante.

Les terres colorantes au sens de la présente invention ne sauraient être substituées par un mélange d'une partie minérale colorante et d'une partie minérale non colorante (i.e. d'un mélange de pigments et de charges, naturels ou synthétiques. Les propriétés intrinsèques des terres colorantes résultent de l'association spécifique entre la partie minérale colorante et la partie minérale non colorante. Cette association spécifique est liée à l'histoire géologique du sol à partir duquel la terre colorante est extraite.

Selon un mode préféré de réalisation, les particules constituant la partie minérale colorante peuvent présenter une taille moyenne, mesurée par microscopie électronique à balayage, inférieur ou égal à 1,7 µm, et en particulier allant de 0,05 µm à 1,7 µm, de préférence de 0,1 µm à 1,5 µm.

### 4. Prélèvement des terres colorantes

Les terres colorantes utilisées dans le cadre de la présente invention peuvent être prélevées dans la nature et subir différents traitements de purification, tels que le lavage, ou le broyage. Elles peuvent être utilisées crues ou calcinées.

On peut éventuellement prévoir, après le prélèvement de la terre et avant les diverses étapes de purification, une étape consistant à séparer les grosses particules récoltées des plus petites, les plus petites étant en général les plus riches en terres colorantes, et les plus grosses étant constituées des minéraux présents dans le sol ou la roche où a été prélevée la terre colorante.

Les terres colorantes peuvent être lavées. Le lavage d'une terre correspond à un procédé nommé lévigation". Selon un procédé de lavage, pendant trente minutes, une quantité de terre est mis à mariner dans trois fois son volume d'eau, dans un récipient fermé. Le couvercle est de préférence distant d'au moins trois centimètres du liquide. Le mélange doit ensuite reposer quelques secondes avant d'être transvasé. Les impuretés et même les gros morceaux argileux doivent être laissés au fond du premier récipient. L'opération peut être répétée en augmentant à chaque fois le temps de marinage. Le mélange est ensuite séché et éventuellement broyé.

La composition selon l'invention peut comprendre de 0,1% à 100% en poids, par rapport au poids de la composition, de terre colorante, de préférence de 1 à 40 % en poids, et plus préférentiellement encore de 1 à 20% en poids.

### Composés naturels additionnels

Outre les terres colorantes décrites précédemment, la composition selon l'invention peut comprendre au moins un autre composé additionnel naturel ou d'origine naturelle.

Au sens de la présente invention, on entend par composés « naturels » :
- les composés d'origine végétale agricole biologique ou végétale sauvage avec un prélèvement raisonné,
- les composés d'origine végétale agricole ou provenant du règne protiste,
- les composés d'origine minérale non fossile,
- les composés d'origine animale, de préférence les composés sécrétés par des animaux (cire d'abeille).

Les composés « d'origine naturelle » sont des composés naturels ayant subits des transformations, ces transformations pouvant être :
1) les transformations décrites précédemment pour les terres colorantes,
2) des procédés visant à extraire, dans le cas d'ingrédients d'origine végétale, une fraction donnée de la plante sans rupture de liaisons chimiques covalentes, ce qui englobe les procédés suivants :
   - l'expression,
   - le pressage,
   - les procédés de Flash détente,
   - la distillation,
   - les procédés d'extraction à l'eau (décoction, infusion, macération),
   - les procédés d'extraction à l'éthanol (dont l'enfleurage),
   - les procédés d'extraction au CO₂ super critique,
   - les procédés d'extraction ci-dessus utilisant le chauffage par micro-ondes,
   - entraînement à la vapeur,
   - les procédés de purification,
   - les procédés de purification basés sur les technologies précédentes,
   - les procédés de purification par passage sur charbon actif, sur oxydes ou sur résine,
   - les procédés thermiques de conservation (appertisation, pasteurisation),
   - les procédés de winterisation ou de frigélisation,
   - les biotransformations appliquées aux matières premières d'origine végétale et catalysées par des organismes génétiquement non modifiés et dont la fonction d'origine correspond à la réaction ciblée,
   - les procédés de conservation par pression (Pascalisation) ou par addition de conservateurs d'origine végétale.
   - Les procédés d'extraction génétique ne rentrent pas dans cette catégorie ainsi que les procédés de conservation par irradiation.
3) des transformations par procédé chimique engendrant une modification mineure, et notamment en ce qui concerne les composés d'origine végétale :
   - extraction par solvant organique (hexane, éthers fluorés, ou autre),
   - hydrolyse,
   - estérification,
   - oxydation utilisant l'oxygène comme oxydant,
   - les hydrogénations d'oléfines,
   - les hydrogénations d'acides et d'esters,
   - les étherifications,
   - la réaction de Guerbet (réaction intermoléculaire entre alcool s'apparentant à un procédé de "cuisson"),
      et pour les ingrédients d'origine minérale non fossile : les procédés permettant d'obtenir des matériaux par dissolution re-précipitation d'espèces minérales conduisant à des oxydes simples ou structurés (zéolites, mésoporeux...),

Selon un mode particulier de réalisation de l'invention, on considère qu'un composé est naturel ou d'origine naturelle lorsqu'il est majoritairement composé de constituants naturels, c'est-à-dire lorsque le rapport pondéral des constituants naturels sur les constituants non naturels qui le composent est supérieur à 1.

A titre d'exemple de matériaux colorants naturels additionnels, on peut citer les terres constituées de composés organiques, telles que notamment les terres bitumineuses comme la terre de Cassel provenant d'Allemagne.

Les compositions de l'invention peuvent comprendre plus de 50% en poids, par rapport au poids total de la composition, de composé naturels ou d'origine naturelle (incluant les terres précédemment citées). En particulier, les compositions selon l'invention peuvent comprendre de 50 à 100 % en poids, par rapport au poids total de la composition, de composé naturels ou d'origine naturelle, de préférence de 70% à 99 % en poids, plus préférentiellement de 80% à 95 % en poids.

Selon un mode préféré de réalisation, les compositions selon l'invention peuvent être constituées exclusivement de composés naturels ou d'origine naturelle.

### Charges matifiantes

La composition selon l'invention comprend au moins une charge dite matifiante.

Au sens de la présente invention, le terme "charge" désigne tout matériau constitué de particules, sphériques ou non, poreuses ou non, non solubles au sein des compositions selon l'invention.

Les charges dites matifiantes sont, de préférence, des charges présentant un indice de réfraction inférieur ou égal à 2,2, notamment inférieur ou égal à 2, en particulier inférieur ou égal à 1,8, et plus particulièrement variant de 1,3 à 1,6.

L'indice de réfraction des particules peut être évalué par la méthode dite d'effacement de contraste. En choisissant deux solvants totalement miscibles d'indices de réfraction relativement éloignés (l'éthanol : 1,36 et alcool phényléthylique : 1,529), il est possible de réaliser des mélanges possédant des indices de réfraction intermédiaires. Les particules en question sont mises en suspension dans ces différents mélanges de solvant et la transparence de ces solutions est ensuite évaluée à l'aide d'un turbidimètre Hach 2100P^{®} commercialisé par la société HACH. L'indice de réfraction de la particule est égal à celui du mélange de solvants pour lequel on obtient la solution la moins turbide, c'est-à-dire présentant le trouble le plus faible et qui correspond à l'écart d'indice de réfraction minimal entre les particules et le mélange de solvant.

Selon un mode de réalisation particulier, les charges matifiantes peuvent avoir une taille particulaire exprimée en volume inférieure à 40 µm, en particulier inférieure à 30 µm, de préférence inférieure à 15 µm. La taille moyenne en volume des charge peut notamment être mesurée par un granulomètre laser, tel que par exemple le Mastersizer 2000^{®} de Malvern et le BI90 + de Broockhaven Instrument Corporation.

D'une manière générale, la composition selon l'invention peut comprendre diverses charges, d'origine minérale ou organique. Ces charges peuvent être de toute forme, notamment plaquettaires, sphériques ou oblongues, quelque soit leur forme cristallographique (par exemple feuillet, cubique, hexagonale, orthorhombique, etc ...). Selon un mode de réalisation particulier, les charges matifiantes sont sphériques.

Selon un autre mode de réalisation particulier, ces charges matifiantes sont également poreuses. Cette porosité peut notamment se traduire par une surface spécifique des particules supérieure à 10 m²/g et en particulier supérieure à 50 m²/g.

Les charges matifiantes considérées selon l'invention confèrent à la composition les comprenant un pouvoir matifiant pouvant être caractérisé à l'aide du protocole suivant.

La composition à tester est étalée à raison de 2 mg/cm² sur une carte de contraste (PRUFKARTE type 24/5-250 cm² commercialisée par la société ERICHSEN) à l'aide d'un tire-film mécanique. La composition est ensuite séchée une nuit à une température de 37 °C préalablement à la mesure de sa réflexion à l'aide d'un gonioréflectomètre commercialisé par la société MICROMODULE. Le résultat obtenu est le rapport R entre la réflexion spéculaire et la réflexion diffuse. La valeur de R est d'autant plus faible que l'effet matifiant procuré par la charge est important.

Dans le cadre de la présente invention, la charge matifiante considérée est généralement choisie de telle manière qu'elle confère à ladite composition un pouvoir matifiant inférieur ou égal à 1, en particulier inférieur ou égal à 0,75 lorsqu'elle est introduite en une teneur de 5% ou moins.

Conviennent tout particulièrement à l'invention, notamment à titre de charges à pouvoir matifiant, les charges choisies parmi :
- les microparticules poreuses de silice, comme par exemple les SILICA BEADS SB150^{®} et SB700^{®} de MIYOSHI de taille moyenne de 5 µm et les SUNSPHERES Série-H^{®} d'ASAHI GLASS comme par exemple les SUNSPHERE H33^{®}, H51^{®} et H53^{®} de tailles respectives de 3, 5 et 5 µm ;
- les poudres de polytétrafluoroéthylène, comme par exemple les PTFE CRI DUST 9205F^{®} de CLARIANT de taille moyenne de 8 µm ;
- tes poudres de résine de silicone comme par exemple les SILICON RESIN TOSPEARL 145A^{®} de GE SILICONE de taille moyenne de 4,5 µm;
- les particules hémisphériques creuses de silicone comme par exemple les NLK 500^{®}, NLK 506^{®} et NLK 510^{®} de TAKEMOTO OIL AND FAT ;
- les poudres de copolymères acryliques, notamment de poly(méth)acrylate de méthyle, comme par exemple les particules PMMA JURIMER MBI^{®} de NIHON JUNYOKI de taille moyenne de 8 µm, les sphères creuses de PMMA vendues sous la dénomination COVABEAD LH85^{®} par la société WACKHERR, et les microsphères de vinylidène/acrylonitrile/méthacrylate de méthylène expansées vendues sous la dénomination EXPANCEL^{®} ;
- les poudres de cire comme les particules PARAFFIN WAX MICROEASE 114S^{®} de MICROPOWDERS de taille moyenne de 7 µm ;
- les poudres de polyéthylène, notamment comprenant au moins un copolymère éthylène/acide acrylique, et en particulier constituées de copolymères éthylène/acide acrylique comme par exemple les particules FLOBEADS EA 209^{®} de SUMITOMO de taille moyenne de 10 µm ;
- les poudres d'organopolysiloxane élastomérique réticulé enrobées de résine de silicone, notamment de résine silsesquioxane, comme décrit par exemple dans le brevet US 5 538 793. De telles poudres d'élastomère sont vendues sous les dénominations
   KSP-100^{®}, KSP-101^{®}, KSP-102^{®}, KSP-103^{®}, KSP-104^{®} et KSP-105^{®} par la société SHIN ETSU;
- les poudres composites de talc/dioxyde de tiane/alumine/silice comme par exemple celles vendues sous la dénomination COVERLEAF AR-80^{®} par la société CATALYST & CHEMICALS ;
- les poudres de polyamide (Nylon^{®}), comme par exemple les particules de Nylon 12 du type ORGASOL d'ATOFINA^{®} de taille moyenne de 10 µm;
- Les microsphères de polyméthacrylate d'allyle / diméthacrylate d'éthylène glycol comme par exemple celles vendues sous la dénomination Polypore E200 par la société AMCOL,
- et leurs mélanges.

Selon un mode préféré de réalisation, la charge matifiante présente des propriétés de soft focus, c'est-à-dire qu'elle diffuse la lumière dans toutes les directions, conférant un effet de flou au support sur lequel elles sont appliquées. Ceci a pour effet de renforcer l'effet matifiant déjà conféré par les charges matifiantes. Les charges matifiantes présentant des propriétés de soft focus sont bien connues de l'art antérieur. Cependant, l'effet de flou obtenu par la présence de particules soft focus est souvent altéré par la présence de charges opaques ou de pigments dans les compositions. Or les inventeurs ont montré qu'en associant des charges matifiantes ayant un effet de soft focus avec des terres colorantes, il était possible de conserver un effet de flou, tout en maintenant une couleur intense de la composition.

Les charges matifiantes soft focus peuvent notamment être choisies parmi :
- les poudres de polytétrafluoroéthylène, comme par exemple les PTFE CRI DUST 9205F^{®} de CLARIANT de taille moyenne de 8 µm;
- les poudres de résine de silicone comme par exemple les SILICON RESIN TOSPEARL 145A^{®} de GE SILICONE de taille moyenne de 4,5 µm;
- les particules hémisphériques creuses de silicone comme par exemple les NLK 500^{®}, NLK 506^{®} et NLK 510^{®} de TAKEMOTO OIL AND FAT ;
- les poudres de copolymères acryliques, notamment de poly(méth)acrylate de méthyle, comme par exemple les particules PMMA JURIMER MBI^{®} de NIHON JUNYOKI de taille moyenne de 8 µm, les sphères creuses de PMMA vendues sous la dénomination COVABEAD LH85^{®} par la société WACKHERR, et les microsphères de vinylidène/acrylonitrile/méthacrylate de méthylène expansées vendues sous la dénomination EXPANCEL^{®} ; - les poudres de cire comme les particules PARAFFIN WAX MICROEASE 114S^{®} de MICROPOWDERS de taille moyenne de 7 µm ;
- les poudres de polyéthylène, notamment comprenant au moins un copolymère éthylène/acide acrylique, et en particulier constituées de copolymères éthylène/acide acrylique comme par exemple les particules FLOBEADS EA 209^{®} de SUMITOMO de taille moyenne de 10 µm ;
- les poudres d'organopolysiloxane élastomérique réticulé enrobées de résine de silicone, notamment de résine silsesquioxane, comme décrit par exemple dans le brevet US 5 538 793. De telles poudres d'élastomère sont vendues sous les dénominations
   KSP-100^{®}, KSP-101^{®}, KSP-102^{®}, KSP-103^{®}, KSP-104^{®} et KSP-105^{®} par la société SHIN ETSU;
- les poudres composites de talc/dioxyde de tiane/alumine/silice comme par exemple celles vendues sous la dénomination COVERLEAF AR-80^{®} par la société CATALYST & CHEMICALS ;
- et leurs mélanges.

D'une manière générale, la charge matifiante peut être présente en une teneur allant de 0,1 à 15 % en poids, par rapport au poids total de la composition, de préférence de 0,5 à 10 % en poids.

### Matières colorantes additionnelles

La composition selon l'invention peut comprendre, outre la terre colorante, au moins une autre matière colorante additionnelle, pour autant que celle-ci n'altère pas l'effet de matité conféré par la charge matifiante.

La matière colorante additionnelle peut être choisie parmi les pigments, nacres et/ou les colorants solubles sous réserve que ceux-ci n'affectent pas les propriétés attendues de la composition.

A titre illustratif et non limitatif des pigments minéraux, on peut plus particulièrement citer les oxydes métalliques jaunes, rouges, bruns comme par exemple les oxydes de fer. Comme poudres métalliques, on peut citer la poudre de cuivre.

Les pigments mis en oeuvre dans le cadre de la présente invention, peuvent être utilisés soit sous leur forme brute ou sous une forme prétraitée, notamment en leur surface.

A titre représentatif de ces traitements de surface, on peut notamment citer celui consistant à traiter le pigment avec un agent hydrophobe et oléofuge de type dérivé phosphate perfluoroalkyle comme décrit dans le brevet EP 1 086 683.

A titre illustratif des pigments convenant plus particulièrement à l'invention, on peut notamment citer les oxydes de fer brun et de fer jaune, enrobés de phosphate de perfluoroalkyle et l'oxyde de titane traité alumine, enrobé de phosphate de perfluoroalkyle, comme en particulier les pâtes pigmentaires commercialisées, sous les dénominations commerciales YELLOW IRON OXYDE COVAFLUOR, PF5 YELLOW 601 (jaune) et PF5 R516L (rouge) par la société DAITO, sous les dénominations commerciales FA50DRF, FA50DYF, FA65DF et FA65DBF par la société KOBO.

Par "nacres", il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique notamment du type précité ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

Les nacres peuvent plus particulièrement posséder une couleur ou un reflet jaune, rose, rouge, bronze, orangé, brun, or et/ou cuivré.

A titre illustratif des nacres pouvant être mises en oeuvre dans le cadre de la présente invention, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna); les nacres de teinte brune notamment commercialisées par la société ENGELHARD sous la dénomination Nu-antique copper 340XB (Cloisonne) et Brown CL4509 (Chromalite); les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

Les colorants solubles peuvent être liposolubles ou hydrosolubles. Ils peuvent être synthétiques ou naturels organiques ou minéraux.

Les colorants liposolubles, synthétiques ou naturels sont, par exemple, le DC Red 17, le DC Red 21, le DC Red 27, le DC Green 6, le DC Yellow 11, le DC Violet 2, le DC Orange 5, le rouge Soudan, les carotènes (le β-carotène, le lycopène), les xanthophylles (capsanthine, capsorubine, lutéine), l'huile de palme, le brun Soudan, le jaune quinoléine, le rocou, le curcumin.

Les colorants hydrosolubles synthétiques ou naturels sont par exemple le FDC Red 4, le DC Red 6, le DC Red 22, le DC Red 28, le DC Red 30, le DC Red 33, le DC Orange 4, le DC Yellow 5, le DC Yellow 6, le DC Yellow 8, le FDC Green 3, le DC Green 5, le FDC Blue 1, la bétanine (betterave), le carmin, la chlorophylline cuivrée, le bleu de méthylène, les anthocyanines (enocianine, carotte noire, hibiscus, sureau), le caramel, la riboflavine.

Les colorants peuvent encore être choisis parmi la juglone, la lawsone, les extraits de soja fermenté, d'algues, de champignons, de micro-organismes. Les sels de Flavylium non substitués en position 3 tels que par exemple ceux décrits dans le brevet EP 1 172 091, les extraits de Gesneria Fulgens, Blechum Procerum, Saxifraga et les pigments susceptibles d'être obtenus par extraction par un solvant organique ou hydro-organique d'un milieu de culture de micromycètes du type monascus Monascus.

### Charges additionnelle

La composition selon l'invention peut comprendre des charges additionnelles, différentes des charges matifiantes décrites précédemment, sous réserve que ceux-ci n'affectent pas les propriétés attendues de la composition.

A titre illustratif des charges additionnelles convenant à l'invention, on peut notamment citer le talc, le mica, le kaolin, la silice colloïdale, la poly-β-alanine, le polyéthylène, la lauroyl-lysine, l'amidon, le nitrure de bore, le carbonate de calcium précipité, le carbonate et l'hydro-carbonate de magnésium, le sulfate de baryum, l'hydroxyhapatite, les microcapsules de verre ou de céramique et les savons métalliques dérivés d'acides organiques carboxyliques ayant de 8 à 22 atomes de carbone, notamment de 12 à 18 atomes de carbone, par exemple le stéarate de zinc, de magnésium ou de lithium, le laurate de zinc, le myristate de magnésium et leurs mélanges.

Pour des raisons évidentes, la quantité de cette charge additionnelle est susceptible de varier significativement selon la nature et/ou la taille particulaire de la charge ou du mélange de charge(s).

D'une manière générale, la charge additionnelle peut être présente à raison de moins de 15 % en poids, notamment à raison de moins de 10 % et en particulier moins de 5 % en poids par rapport au poids total de la composition.

### Milieu Physiologiquement acceptable

La composition selon l'invention comprend un milieu physiologiquement acceptable.

Par "milieu physiologiquement acceptable", on désigne un milieu non toxique et susceptible d'être appliqué sur la peau d'êtres humains. Le milieu physiologiquement acceptable est généralement adapté à la nature de la peau sur laquelle doit être appliquée la composition ainsi qu'à la forme sous laquelle la composition est destinée à être conditionnée, notamment fluide à la température ambiante et sous pression atmosphérique.

### Phase aqueuse

La composition selon l'invention peut comprendre au moins une phase aqueuse.

La phase aqueuse comprend notamment de l'eau.

Elle peut également comprendre un mélange d'eau et de solvant organique miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, et les aldéhydes en C₂-C₄.

A titre d'exemple de monoalcool convenant à la mise en oeuvre de l'invention, on peut mentionner les composés hydrocarbonés saturés comprenant une unique fonction hydroxyle et comprenant de 2 à 15 atomes de carbone, en particulier de 4 à 12 atomes de carbone.

A titre d'exemple de monoalcool convenant à l'invention, on peut citer, de manière non limitative l'éthanol, le propanol, l'isopropanol, le butanol, le pentanol, le propanol, l'hexanol, l'heptanol, l'octanol, le décanol et le dodécanol.

Par le terme "solvant polyhydroxylé", on entend couvrir tout composé hydrocarboné comprenant au moins deux fonctions hydroxyle et comprenant de 4 à 40 atomes de carbone, en particulier de 6 à 36 atomes de carbone, en particulier de 8 à 32 atomes de carbone, en particulier de 16 à 28 atomes de carbone, et plus particulièrement de 18 à 24 atomes de carbone.

Les chaînes hydrocarbonées peuvent, le cas échéant, être interrompues par la présence d'au moins un hétéroatome, et notamment un atome d'oxygène.

Le polyol convenant à la mise en oeuvre de la présente invention peut être, notamment, choisi parmi les alcools linéaires, ramifiés, cycliques ou polycycliques, saturés ou insaturés.

Ainsi, le polyol peut être choisi par exemple parmi un diol, un triol, un tétraol, ou un pentaol, ou un de leurs esters.

Le polyol peut être un diol, ou un de ses esters, par exemple choisi parmi un dimère d'alcool gras, un mono- ou poly-glycérol, un mono- ou poly-alkylène en C₂₋₄ glycol, le 1,4 butanediol, et le pentaérythritol.

A titre d'exemple de diol pouvant également convenir à la mise en oeuvre de l'invention, on peut citer, de manière non exhaustive, le butanediol, le pentanediol, le propanediol, l'hexanediol, l'hexylèneglycol, l'heptanediol, l'octanediol, le nonanediol, le décanediol, l'un-décanediol, le dodécanediol, le tridécanediol, le tétradécanediol, le pentadécanediol, l'hexadécanediol, le nonadécanediol, l'octadecènediol, le cyclohexanediol, le 1,1'-oxydipropanediol le diglycérol, l'érythritol, le pentaérythritol, le xylitol, le sorbitol, le phytantriol (3,7,11,15-tetraméthyl-1,2,3-trihydroxy-hexadecane) l'éthylèneglycol, le xylèneglycol et leurs isomères.

Convient tout particulièrement comme mélange, un mélange à base au moins d'éthanol et de propylène glycol ou de dipropylène glycol ou de butylène glycol.

La phase aqueuse (eau et éventuellement le(s) solvant(s) organique(s) miscible(s) à l'eau) peut être présente, à une teneur allant de 1 % à 99 % en poids, notamment allant de 3 % à 80 % en poids, et en particulier allant de 5 % à 60 %, en poids par rapport au poids total de la composition considérée.

### Phase grasse

La composition selon l'invention peut comprendre une phase grasse et notamment au moins un corps gras liquide à température ambiante (25 °C) et/ou un corps gras solide à température ambiante tel que les cires, les corps gras pâteux, les gommes et leurs mélanges.

La phase grasse de la composition selon l'invention peut notamment comprendre, à titre de corps gras liquide, au moins une huile volatile ou non volatile ou un de leurs mélanges.

Par "huile volatile", on entend au sens de l'invention toute huile susceptible de s'évaporer au contact de la peau en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,01 à 300 mm de Hg (1,33 Pa à 40.000 Pa) et de préférence supérieure à 0,3 mm de Hg (30 Pa).

Par "huile non volatile", on entend une huile restant sur la peau à température ambiante et pression atmosphérique au moins plusieurs heures et ayant notamment une pression de vapeur inférieure à 0,01 mm de Hg (1,33 Pa).

Ces huiles volatiles ou non volatiles peuvent être des huiles hydrocarbonées, des huiles siliconées, ou leurs mélanges. On entend par "huile hydrocarbonée", une huile contenant principalement des atomes d'hydrogène et de carbone et éventuellement des atomes d'oxygène, d'azote, de soufre, de phosphore.

Les huiles hydrocarbonées volatiles peuvent être choisies parmi les huiles hydrocarbonées ayant de 8 à 16 atomes de carbone, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars^{®} ou de Permetyls^{®}, les esters ramifiés en C₈-C₁₆ tels que le néopentanoate d'iso-hexyle, et leurs mélanges. D'autres huiles hydrocarbonées volatiles comme les distillats de pétrole, notamment ceux vendus sous la dénomination Shell Solt^{®} par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité ≤ 8 centistokes (8 x 10⁻⁶ m²/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile peut être présente dans une composition selon l'invention à une teneur allant de 0,1 à 98 % en poids, notamment de 1 % à 65 % en poids, et en particulier de 1 % à 50 % en poids, par rapport au poids total de la composition.

Les huiles non volatiles peuvent notamment être choisies parmi les huiles hydrocarbonées fluorées et/ou siliconées non volatiles.

Comme huile hydrocarbonée non volatile, on peut notamment citer :
- les huiles hydrocarbonëes d'origine animale,
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810, 812 et 818^{®} par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges,
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, les benzoates d'alcools en C₁₂ à C₁₅, le laurate d'hexyle, l'adipate de diisopropyle, l'isononanoate d'isononyle, le palmitate de 2-éthyl-hexyle, l'isostéarate d'isostéaryle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol,
- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates.

Les huiles non volatiles peuvent être présentes dans une composition selon l'invention en une teneur allant de 0,01 à 90 % en poids, notamment de 0,1 à 85 % en poids, et en particulier de 1 % à 70 % en poids, par rapport au poids total de la composition.

Plus généralement, le corps gras liquide peut être présent à raison de 0,01 à 90 % en poids et notamment de 0,1 à 85 % en poids, par rapport au poids de la phase grasse.

En ce qui concerne le corps gras solide à température ambiante et à pression atmosphérique, il peut être choisi parmi les cires, les corps gras pâteux, les gommes et leurs mélanges. Ce corps gras solide peut être présent à raison de 0,01 à 50 %, notamment de 0,1 à 40 % et en particulier de 0,2 à 30 % en poids, par rapport au poids total de la phase grasse.

Ainsi, une composition selon l'invention peut comprendre au moins un composé gras pâteux à température ambiante.

Par "corps gras pâteux" au sens de l'invention, on entend des corps gras ayant un point de fusion allant de 20 à 55 °C, de préférence 25 à 45 °C, et/ou une viscosité à 40 °C allant de 0,1 à 40 Pa.s (1 à 400 poises), de préférence 0,5 à 25 Pa.s, mesurée au Contraves TV ou Rhéomat 80, équipé d'un mobile tournant à 60 Hz. L'homme du métier peut choisir le mobile permettant de mesurer la viscosité, parmi les mobiles MS-r3 et MS-r4, sur la base de ses connaissances générales, de manière à pouvoir réaliser la mesure du composé pâteux testé.

De préférence, ces corps gras sont des composés hydrocarbonés, éventuellement de type polymérique ; ils peuvent également être choisis parmi les composés siliconés; ils peuvent aussi se présenter sous forme d'un mélange de composés hydrocarbonés et/ou siliconés. Dans le cas d'un mélange de différents corps gras pâteux, on utilise de préférence les composés pâteux hydrocarbonés (contenant principalement des atomes de carbone et d'hydrogène et éventuellement des groupements ester), en proportion majoritaire.

Parmi les composés pâteux susceptibles d'être utilisés dans une composition selon l'invention, on peut citer les lanolines et les dérivés de lanoline comme les lanolines acétylées, les lanolines oxypropylènées ou le lanolate d'isopropyle, ayant une viscosité de 18 à 21 Pa.s, de préférence 19 à 20,5 Pa.s, et/ou un point de fusion de 30 à 55 °C et leurs mélanges. On peut également utiliser des esters d'acides ou d'alcools gras, notamment ceux ayant 20 à 65 atomes de carbone (point de fusion de l'ordre de 20 à 35 °C et/ou viscosité à 40 °C allant de 0,1 à 40 Pa.s) comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux comme l'acide poly(12-hydroxystéarique) et leurs mélanges. Comme triglycéride d'origine végétale, on peut utiliser les dérivés d'huile de ricin hydrogénée, tels que le "THIXINR" de Rheox.

On peut aussi citer les corps gras pâteux siliconés tels que les polydiméthylsiloxanes (PDMS) de hauts poids moléculaires et en particulier ceux ayant des chaînes pendantes du type alkyle ou alcoxy ayant de 8 à 24 atomes de carbone, et un point de fusion de 20-55 °C, comme les stéaryl diméthicones notamment ceux vendus par la société Dow Corning sous les noms commerciaux de DC2503^{®} et DC25514^{®}, et leurs mélanges.

Le corps gras pâteux peut être présent dans une composition selon l'invention en une teneur allant de 0,01 à 50 % en poids, de préférence allant de 0,1 à 45 % en poids, et mieux allant de 0,2 % à 30 % en poids, par rapport au poids total de ladite composition.

La composition selon l'invention peut comprendre en outre une cire. La cire peut être solide à température ambiante (25 °C), à changement d'état solide/liquide réversible, ayant une température de fusion supérieure à 30 °C pouvant aller jusqu'à 200 °C, une dureté supérieure à 0,5MPa et présentant à l'état solide une organisation cristalline anisotrope. Elle peut être hydrocarbonée, fluorée et/ou siliconée et être d'origine animale, végétale, minérale ou synthétique. Elle peut être choisie par exemple parmi la cire d'abeille, la cire de Carnauba, la cire de Candellila, les cires de paraffine, l'huile de ricin hydrogénée, les cires de silicone, les cires microcristallines, et leurs mélanges.

En particulier, la cire peut être présente sous forme d'émulsion cire-dans-eau.

La cire peut être présente dans une composition selon l'invention en une teneur allant de 0,01 % à 50 % en poids, en particulier de 0,1 % à 30 % en poids, et notamment de 0,2 % à 20 % en poids, par rapport au poids total de la composition.

### Elastomères de silicone

La composition selon l'invention comprend au moins un élastomère de silicone qui peut être non émulsionnant ou émulsionnant.

Le terme élastomères de silicone "non émulsionnant" définit des élastomères organopolysiloxane ne contenant pas de chaîne hydrophile telle que motifs polyoxyalkylènes ou polyglycérolés.

L'élastomère de silicone non émulsionnant est un organopolysiloxane réticulé élastomère pouvant être obtenu par réaction d'addition réticulation de diorganopolysiloxane contenant au moins un hydrogène lié au silicium et de diorganopolysiloxane ayant des groupements à insaturation éthylénique liés au silicium, notamment en présence de catalyseur platine ; ou par réaction de condensation réticulation déhydrogénation entre un diorganopolysiloxane à terminaisons hydroxyle et un diorganopolysiloxane contenant au moins un hydrogène lié au silicium, notamment en présence d'un organoétain ; ou par réaction de condensation réticulation d'un diorganopolysiloxane à terminaisons hydroxyle et d'un organopolysilane hydrolysable ; ou par réticulation thermique d'organopolysiloxane, notamment en présence de catalyseur organopéroxyde ; ou par réticulation d'organopolysiloxane par radiations de haute énergie telles que rayons gamma, rayons ultraviolet, faisceau électronique.

De préférence, l'organopolysiloxane réticulé élastomère est obtenu par réaction d'addition réticulation (A2) de diorganopolysiloxane contenant au moins deux hydrogènes liés chacun à un silicium, et (B2) de diorganopolysiloxane ayant au moins deux groupements à insaturation éthylénique liés au silicium, notamment en présence (C2) de catalyseur platine, comme par exemple décrit dans la demande EP-A-295886.

En particulier, l'organopolysiloxane peut être obtenu par réaction de diméthylpolysiloxane à terminaisons diméthylvinylsiloxy et de méthylhydrogénopolysiloxane à terminaisons triméthylsiloxy, en présence de catalyseur platine.

Le composé (A2) est le réactif de base pour la formation d'organopolysiloxane élastomère et la réticulation s'effectue par réaction d'addition du composé (A2) avec le composé (B2) en présence du catalyseur (C2).

Le composé (A2) est avantageusement un diorganopolysiloxane ayant au moins deux groupes alkényles inférieur (par exemple en C2-C4) ; le groupe alkényle inférieur peut être choisi parmi les groupes vinyl, allyl, et propényl. Ces groupements alkényles inférieurs peuvent être situés en toute position de la molécule organopolysiloxane mais sont de préférence situés aux extrémités de la molécule organopolysiloxane. L'organopolysiloxane (A2) peut avoir une structure chaîne ramifiée, chaîne linéaire, cyclique ou réseau mais la structure chaîne linéaire est préférée. Le composé (A2) peut avoir une viscosité allant de l'état liquide à l'état de gomme. De préférence, le composé (A2) a une viscosité d'au moins 100 centistokes à 25 °C.

Les organopolysiloxanes (A2) peuvent être choisi parmi les méthylvinylsiloxanes, les copolymères méthylvinylsiloxane-diméthylsiloxanes, les diméthylpolysiloxanes à terminaisons diméthylvinylsiloxy, les copolymères diméthylsiloxane-méthylphénylsiloxane à terminaisons diméthylvinylsiloxy, les copolymères diméthylsiloxane-diphénylsiloxane-méthylvinylsiloxane à terminaisons diméthylvinylsiloxy, les copolymères diméthylsiloxane-méthylvinylsiloxane à terminaisons triméthylsiloxy, les copolymères diméthylsiloxane-méthylphénylsiloxane-méthylvinylsiloxane à terminaisons triméthylsiloxy, les méthyl(3,3,3-trifluoropropyl)polysiloxane à terminaisons diméthylvinylsiloxy, et les copolymères diméthylsiloxane-méthyl(3,3,3-trifluoropropyl)siloxane à terminaisons diméthylvinylsiloxy.

Le composé (B2) est en particulier un organopolysiloxane ayant au moins 2 hydrogènes liés au silicium dans chaque molécule et est donc le réticulant du composé (A2). Avantageusement, la somme du nombre de groupements éthyléniques par molécule du composé (A2) et le nombre d'atomes d'hydrogène liés au silicum par molécule du composé (B2) est d'au moins 4.

Le composé (B2) peut être sous toute structure moléculaire, notamment de structure chaîne linéaire, chaîne ramifiée, structure cyclique.

Le composé (B2) peut avoir une viscosité à 25 °C allant de 1 à 50 000 centistokes, notamment pour être bien miscible avec le composé (A).

Il est avantageux que le composé (B2) soit ajouté en une quantité telle que le rapport moléculaire entre la quantité totale d'atomes d'hydrogène liés au silicium dans le composé (B2) et la quantité totale de tous les goupements à insaturation éthylénique dans le composé (A2) aille dans la gamme de 1/1 à 20/1.

Le composé (B2) peut être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane.

Le composé (C2) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C2) est de préférence ajouté de 0,1 à 1000 parts en poids, mieux de 1 à 100 parts en poids, en tant que métal platine propre pour 1000 parts en poids de la quantité totale des composés (A2) et (B2).

D'autres groupes organiques peuvent être liés au silicium dans les organopolysiloxane (A2) et (B2) décrits précédemment, comme par exemple des groupes alkyles tels que méthyl, éthyl, propyl, butyl, octyl ; des groupes alkyles substitués tels que 2-phényléthyl, 2-phénylpropyl, 3,3,3-trifluoropropyl ; des groupes aryles tels que phényl, tolyl, xylyl ; des groupes aryles substitutés tel que phényléthyl ; et des groupes hydrocarbonés monovalents substitués tels que un groupe époxy, un gropue ester carboxylate, un groupe mercapto.

Selon un mode préféré de réalisation, l'élastomère de silicone non émulsionnant est mélangé avec au moins une huile hydrocarbonée et/ou une huile siliconée pour former un gel. Dans ces gels, l'élastomère non émulsionnant est sous forme de particules non-sphériques.

Comme élastomères non émulsionnants, on peut utiliser ceux vendus sous les dénominations "KSG-6", "KSG-15", "KSG-16", "KSG-18", "KSG-31", "KSG-32", "KSG-33", "KSG-41", "KSG-42", "KSG-43", "KSG-44" par la société Shin Etsu, "DC 9040", "DC9041", "DC 9509", "DC9505", "DC 9506" par la société Dow Corning, "GRANSIL" par la société Grant Industries, "SFE 839" par la société General Electric.

L'élastomère de silicone non sphérique peut être émulsionnant.

Par élastomère de silicone émulsionnant on entend un élastomère de silicone comprenant au moins une chaîne hydrophile.

L'élastomère de silicone émulsionnant peut être choisi parmi les élastomères de silicone polyoxyalkylénés.

L'élastomère de silicone polyoxyalkyléné est un organopolysiloxane réticulé pouvant être obtenu par réaction d'addition réticulation de diorganopolysiloxane contenant au moins un hydrogène lié au silicium et d'un polyoxyalkylène ayant au moins deux groupements à insaturation éthylénique.

De préférence, l'organopolysiloxane réticulé polyoxyalkyléné est obtenu par réaction d'addition réticulation (A1) de diorganopolysiloxane contenant au moins deux hydrogènes liés chacun à un silicium, et (B1) de polyoxyalkylène ayant au moins deux groupements à insaturation éthylénique, notamment en présence (C1) de catalyseur platine, comme par exemple décrit dans les brevets US5236986 et US5412004.

En particulier, l'organopolysiloxane peut être obtenu par réaction de polyoxyalkylène (notamment polyoxyéthyléne et/ou polyoxypropylène) à terminaisons diméthylvinylsiloxy et de méthylhydrogénopolysiloxane à terminaisons triméthylsiloxy, en présence de catalyseur platine.

Les groupes organiques liés aux atomes de silicium du composé (A1) peuvent être des groupes alkyles ayant de 1 à 18 atomes de carbone, tels que méthyl, éthyl, propyl, butyl, octyl, décyl, dodécyl (ou lauryl), myristyl, cétyl, stéaryl ; des groupes alkyles substitués tels que 2-phényléthyl. 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto.

Le composé (A1) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane, les copolymères diméthylsiloxane-méthylhydrogénosiloxane-laurylméthylsiloxane à terminaisons triméthylsiloxy.

Le composé (C1) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Avantageusement, les élastomères de silicone polyoxyalkylénés peuvent être formés à partir de composés divinyliques, en particulier des polyoxyalkylènes ayant au moins deux groupes vinyliques, réagissant avec des liaisons Si-H d'un polysiloxane.

L'élastomère de silicone polyoxyalkyléné selon l'invention est véhiculé sous forme de gel dans au moins une huile hydrocarbonée et/ou une huile siliconée. Dans ces gels, l'élastomère polyoxyalkyléné est sous forme de particules non-sphériques.

Des élastomères polyoxyalkylénés sont notamment décrits dans les brevets US5236986, US5412004, US5837793, US5811487 dont le contenu est incorporé par référence.

Comme élastomère de silicone polyoxyalkyléné, on peut utiliser ceux commercialisés sous les dénominations "KSG-21", "KSG-20", "KSG-30", "KSG-31", KSG-32", "KSG-33", "KSG-210", "KSG-310", "KSG-320", "KSG-330", "KSG-340", "X-226146" par la société Shin Etsu, "DC9010", "DC9011" par la société Dow Corning.

L'élastomère de silicone peut également se présenter sous la forme de poudre d'élastomère de silicone. La poudre d'élastomère de silicone peut notamment être sphérique.

Avantageusement, l'élastomère de silicone sous forme de poudre est non émulsionnant. Il peut notamment être obtenu par les procédés de synthèse des élastomères non émulsionnants décrits précédemment.

Des organopolysiloxanes élastomères sphériques sont notamment décrits dans les demandes JP-A-61-194009, EP-A-242219, EP-A-295886, EP-A-765656, dont le contenu est incorporé à titre de référence.

Comme poudres d'organopolysiloxanes élastomères, on peut utiliser celles vendues sous les dénominations "Dow Corning 9505 Powder", "Dow Corning 9506 Powder" par la société Dow Corning décrites précédemment, ou encore celles décrites dans le document JP-A-02-243612, telles que celles vendues sous la dénomination « TREFIL POWDER E-506C » par la société DOW CORNING.

La présence dans la composition d'un élastomère de silicone tel que décrit précédemment peut notamment permettre d'améliorer encore les propriétés de matité et de soft focus de la composition.

L'élastomère de silicone peut être présent dans la composition selon l'invention en une teneur en matière active allant de 0,01 % à 8 % en poids, par rapport au poids total de la composition, de préférence allant de 2 % à 7 % en poids, et plus préférentiellement allant de 3 % à 6 % en poids.

### Polymère filmogène

La composition selon l'invention peut comprendre, en outre, au moins un polymère filmogène.

Selon la présente invention, on entend par "polymère filmogène", un polymère apte à former à lui seul ou en présence d'un agent auxiliaire de filmification, un film continu et adhérent sur la peau.

On utilise de préférence un polymère filmogène apte à former un film hydrophobe, c'est-à-dire un polymère dont le film a une solubilité dans l'eau à 25 °C inférieure à 1 % en poids.

Le polymère filmogène peut notamment être au moins un polymère choisi parmi le groupe comprenant :
- les polymères filmogènes hydrosolubles,
- des dispersions aqueuses de particules de polymères filmogènes hydrodispersibles, encore appelées "latex" ; dans ce cas, la composition doit comprendre une phase aqueuse,
- des polymères filmogènes liposolubles,
- les polymères filmogènes lipodispersibles sous forme de dispersions non aqueuses de particules de polymère, de préférence des dispersions de particules polymériques, le cas échéant stabilisées en leur surface par au moins un agent stabilisant, dans une ou plusieurs huiles de silicones et/ou.

Le polymère filmogène peut être présent dans une composition selon l'invention en une teneur en matières sèches allant de 0,01 % à 20 % en poids par rapport au poids total de la composition et notamment de 0,5 % à 10 % en poids.

Parmi les polymères filmogènes utilisables selon l'invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats). Les polymères filmogènes de type radicalaire peuvent être notamment des polymères ou des copolymères, vinyliques, notamment des polymères acryliques.

Comme dispersion aqueuse de polymère filmogène, on peut utiliser les dispersions acryliques vendues sous les dénominations NEOCRYL XK-90^{®}, NEOCRYL A-1070^{®}, NEOCRYL A-1090^{®}, NEOCRYL BT-62^{®}, NEOCRYL A-1079^{®}, NEOCRYL A-523^{®} par la société AVECIA-NEORESINS, DOW LATEX 432^{®} par la société DOW CHEMICAL, DAITOSOL 5000 AD^{®} par la société DAITO KASEY KOGYO; ou bien encore les dispersions aqueuses de polyuréthane vendues sous les dénominations NEOREZ R-981 ^{®}, NEOREZ R-974^{®} par la société AVECIA-NEORESINS, les AVALURE UR-405^{®}, AVALURE UR-410^{®}, AVALURE UR-425^{®}, AVALURE UR-450^{®}, SANCURE 875^{®}, SANCURE 861^{®}, SANCURE 878^{®}, SANCURE 2060^{®} par la société GOODRICH; IMPRANIL 85^{®} par la société BAYER, AQUAMERE H-1511^{®} par la société HYDROMER.

Comme exemples de polymères filmogènes hydrosolubles, on peut citer les protéines, les polymères de chitine ou de chitosane anioniques, cationiques, amphotères ou non-ioniques, les polymères de cellulose, les polymères ou copolymères acryliques, les polymères vinyliques, les polymères d'origine naturelle, éventuellement modifiés, et leurs mélanges.

Le polymère filmogène peut être également présent sous forme de particules, stabilisées en surface, dispersées dans la phase grasse liquide. Des dispersions de polymère filmogène dans la phase grasse liquide, en présence d'agents stabilisants, sont notamment décrites dans les documents EP-A-749746, EP-A-923928, EP-A-930060.

Comme polymères filmogènes liposolubles utilisables dans l'invention, on peut également citer les polyalkylènes et notamment les copolymères d'alcènes en C₂-C₂₀, comme le polybutène, les alkylcelluloses avec un radical alkyle linéaire ou ramifié, saturé ou non en C₁ à C₈ comme l'éthylcellulose et la propylcellulose, les copolymères de la vinylpyrolidone (VP) et notamment les copolymères de la vinylpyrrolidone et d'alcène en C₂ à C₄₀ et mieux en C₃ à C₂₀. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

La composition selon l'invention peut, en outre, comprendre un agent auxiliaire de filmification favorisant la formation d'un film avec le polymère filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

### Actifs

La composition cosmétique selon l'invention peut également comprendre des agents actifs habituels dans le domaine cosmétique ou dermatologique.

On peut citer en particulier tous les actifs connus pour leur activité sur le vieillissement de la peau comme les agents keratolytiques ou prodesquamants, par exemple les α-hydroxy-acides, les β-hydroxy-acides, les α -céto-acides, les β -ceto-acides, les rétinoïdes et leurs esters, le rétinal, l'acide rétinoïque et ses dérivés. On peut citer aussi les vitamines, telles que par exemple les vitamines B3 ou PP, B5, E, K1, et les dérivés de ces vitamines et notamment leurs esters; les agents anti-radicaux libres; les filtres solaires; les agents hydratants comme les polyols; les céramides; la DHEA et ses dérivés; le coenzyme Q10; les agents blanchissants et dépigmentants comme l'acide kojique, la vitamine C et ses dérivés, les dérivés de para-aminophénols, l'arbutine et leurs dérivés, l'adénosine et ses dérivés, les c-glycosides (proxylane) et leur dérivés et leurs mélanges.

### Ingrédients cosmétiques additionnels

La composition peut comprendre d'autres ingrédients cosmétiques usuels pouvant être choisis notamment parmi les parfums, les conservateurs, les filtres solaires physiques et chimiques, les séquestrants, les actifs liposolubles ou hydrosolubles, les hydratants tels que les polyols et notamment la glycérine, des ajusteurs de pH (acides ou bases).

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

### Galénique

La composition de soin ou de maquillage peut notamment une composition non rincée.

La composition considérée selon l'invention se présente généralement sous la forme d'une composition d'une crème, d'un fond de teint notamment à appliquer sur le visage ou le cou, ou base de maquillage pour le visage ou d'une composition de maquillage pour le corps.

La composition selon l'invention peut se présenter sous la forme d'un fluide par exemple pâteux ou liquide. Elle peut se présenter sous forme de pâte souple, d'un onguent, d'une pommade solide ou fluide de type crème. Par exemple, elle peut être une émulsion huile-dans-eau, eau-dans-huile ou multiple, une émulsion solide notamment de type eau dans huile, un gel notamment anhydre, solide ou souple et même sous forme biphasique.

Selon un mode préféré de réalisation, la composition selon l'invention se présente sous forme d'émulsion ou de poudre, libre ou compactée.

L'invention pourra être mieux comprise à la lecture de la description détaillée qui va suivre, d'exemples de mise en oeuvre non limitatifs de celle-ci.

L'invention est illustrée plus en détails par les exemples décrits ci-après.

### Exemple 1 et 2 comparatifs

On a préparé deux compositions de soin ayant la formule suivante :

| | | Exemple 1 (comparatif) | Exemple 2 |
|---|---|---|---|
| *A-* | Mélange de mono et di-stéarate de glycéryle / stéarate de polyéthylène glycol (100 OE) vendu sous la dénomination commerciale Arlacel 165FI par la société Uniquema | 2.00 g | 2.00 g |
| | | | |
| | Mélange de tatrate de di-alkyle linéaire en C14-C15, d'alcool cétylstéarilique et d'alcool laurylique oxyéthyléné (25 OE) oxypropyléné (25 OP) vendu sous la dénomination commerciale Cosmacol PSE par la société Sasol | 1.50 g | 1.50 g |
| | | | |
| | Cyclohexasiloxane | 10.00 g | 10.00 g |
| | Alcool Stéarylique | 1.00 g | 1.00 g |
| *B*- | Eau | 81.75 g | 73.75 g |
| | Phenoxyethanol | 1.00 g | 1.00 g |
| | Sel d'acide éthylène diamine tétracétique (EDTA) | 0.05 g | 0.05 g |
| | Copolymère d'AMPS réticulé vendu sous la dénomination commerciale Hostacerin AMPS par la société Clariant | 0.40 g | 0.40 g |
| | Gomme de xanthane | 0.20 g | 0.20 g |
| *C-* | Terre colorante rouge (rouge ERCOLANO) | 2,00 g | 2.00 g |
| | Particules hémisphériques creuses de silicone vendues sous la dénomination commerciale NLK 506^{®} par la société TAKEMOTO OIL AND FAT | | 3.00 g |

### Mode opératoire :

On chauffe la phase B à environ 75°C et y incorporer le copolymère d'AMPS réticulé; agiter jusqu'à obtention d'un gel homogène.

On chauffe la phase A à environ 75°C puis on réalise l'émulsion en incorporant la phase A dans la phase B sous agitation.

On abaisse la température à 40-45°C, puis on incorpore la phase C et on maintient l'agitation jusqu'à refroidissement complet.

Les compositions obtenues sont appliquées sur la peau du visage. Le dépôt obtenu avec la composition 2 est mat et homogène.

### Mise en évidence de l'effet d'homogénéisation du teint des compositions selon l'invention

Les formules selon l'invention procurent à la peau un effet homogénéisant.

Au sens de l'invention, ceci désigne le fait que les formules telles que décrit ci après provoquent après application sur la peau un écart dé couleur caractérisé par une coloration équilibré entre le jaune et le rouge.

Pour ceci, on étale la composition sur une zone de l'avant bras (6.25 cm²) à raison de 2mg/cm². On mesure ensuite l'écart de couleur ΔE entre la zone traitée et la zone non traitée avec un colorimètre Minolta CR - 400. L'écart de couleur est obtenu à l'aide de la formule d'écart de couleur de Hunter dans l'espace colorimétrique L, a, b : ΔE = [(ΔL)² + (Δa)² + (Δb)²]^{0.5}.

On défini une coloration équilibrée entre le jaune et le rouge par le rapport Δa / Δb comme étant le plus proche possible de 1. Un tel rapport Δa/Δb assure un apport de couleur à la peau qui permet une homogénéisation naturelle du teint de la peau.

Dans l'expérience qui suit on a appliqué selon le protocole décrit les compositions 1et 2. Les résultats mesurés sont donné ci-joint :

| | Δa | Δb | Δa / Δb | ΔE |
|---|---|---|---|---|
| Exemple 1 émulsion contenant 2% de terres colorantes rouges - | 4.4 | 3 | 1.46 | 6 |
| Exemple 2 émulsion contenant 2% de terres colorantes rouges et 3% de particules NLK-506 | 3 | 3 | 1 | 5 |

Ces résultats mettent en évidence un effet homogénéisant de l'association terres colorantes rouges et charges matifiantes (Δa / Δb = 1), par rapport à une composition comprenant des terres colorantes seules (Δa / Δb = 1.46).

### Exemple 3 : composition cosmétique

On a préparé une composition de soin ayant la formule suivante :

| | | Exemple 3 |
|---|---|---|
| *A-* | Mélange de mono et di-stéarate de glycéryle / stéarate de polyéthylène glycol (100 OE) vendu sous la dénomination commerciale Arlacel 165FI par la société Uniquema | 2.00 g |
| | | |
| | Mélange de tatrate de di-alkyle linéaire en C14-C15. d'alcool cétylstéarilique et d'alcool laurytique oxyéthyléné (25 OE) oxypropyléné (25 OP) vendu sous la dénomination commerciale Cosmacol PSE par la société Sasol | 1.50 g |
| | | |
| | Cyclohexasiloxane | 10.00 g |
| | Alcool Stéarylique | 1.00 g |
| *B*- | Eau | 69.75 g |
| | Phenoxyethanol | 1.00 g |
| | Sel d'acide éthylène diamine tétracétique | 0.05 g |
| | (EDTA) | |
| | | |
| | Copolymère d'AMPS réticulé vendu sous la dénomination commerciale Hostacerin AMPS par la société Clariant | 0.40 g |
| | | |
| | Gomme de xanthane | 0.20 g |
| | | |
| *C-* | Terre colorante rouge (rouge ERCOLANO) | 2.00 g |
| | Particules hémisphériques creuses de silicone vendues sous la dénomination commerciale NLK 506^{®} par la société TAKEMOTO OIL AND FAT | 3.00 g |
| | Nacre mica/oxyde de titane/oxyde d'étain | 1.00 g |

### Mode opératoire :

Le mode opératoire est la même que pour les exemples 1 et 2.

La composition obtenue est appliquée sur la peau du visage. Le dépôt est mat et homogène.

## Revendications

1. Composition cosmétique comprenant, dans un milieu physiologiquement acceptable, au moins une particule composite autrement nommée terre colorante, existant à l'état naturel, comprenant une partie minérale colorante et une partie minérale non colorante, et au moins une charge matifiante.

2. Composition selon la revendication précédente, **caractérisée en ce que** les terres colorantes sont des particules composites comprenant une partie minérale colorante comprenant du fer, de préférence sous forme oxydée.

3. Composition selon l'une quelconque des revendications précédentes, **caractérisée en ce que** les terres colorantes comprennent des oxydes métalliques colorés autres que l'oxyde de fer, tels que l'oxyde de manganèse ou l'oxyde d'aluminium.

4. Composition selon l'une quelconque des revendications précédentes, **caractérisée en ce que** les terres colorantes sont choisies parmi les terres jaunes, les terres rouges, les terres vertes, les terres brunes ou terres d'ombre et leur mélange.

5. Composition selon l'une quelconque des revendications précédentes, **caractérisée en ce que** la terre colorante comprend une partie minérale non colorante choisie parmi l'argile, du gypse, la calcite, le carbonate de magnésium, le quartz, l'alumine hydratée, et leurs mélanges.

6. Composition selon l'une quelconque des revendications précédentes, **caractérisée en ce que** les terres colorantes comprennent au moins une partie minérale colorante et au moins une partie minérale non colorante, la partie minérale colorante étant solidaires avec l'un au moins des constituants de la partie minérale non colorant.

7. Composition selon l'une quelconque des revendications précédentes, **caractérisée en ce que** la charge matifiante possède un indice de réfraction inférieur ou égal à 2,2, notamment inférieur ou égal à 2, en particulier inférieur ou égal à 1,8 et plus particulièrement variant de 1,3 à 1,6.

8. Composition selon l'une quelconque des revendications précédentes, **caractérisée en ce que** la charge matifiante possède une taille particulaire moyenne en volume inférieure à 40 µm, de préférence inférieure à 30 µm et plus préférentiellement inférieure à 20 µm.

9. Composition selon l'une quelconque des revendications précédentes, **caractérisée en ce que** la charge matifiante confère à ladite composition un pouvoir matifiant inférieur à 0,75.

10. Composition selon l'une quelconque des revendications précédentes, **caractérisée en ce que** la charge matifiante possède une surface spécifique des particules supérieure à 10 m²/g et en particulier supérieure à 50 m²/g.

11. Composition selon l'une quelconque des revendications précédentes, **caractérisée en ce que** la charge matifiante est choisie parmi
- les microparticules poreuses de silice,
- les poudres de polytétrafluoroéthylène,
- les poudres de résine de silicone,
- les particules hémisphériques creuses de silicone,
- les poudres de copolymères acryliques,
- les poudres de cire,
- les poudres de polyéthylène,
- les poudres d'organopolysiloxane élastomérique réticulé enrobées de résine de silicone,
- les poudres composites de talc/dioxyde de tiane/alumine/silice,
- les poudres de polyamide, et
- leurs mélanges.

12. Composition selon l'une quelconque des revendications précédentes, **caractérisée en ce que** la charge matifiante est présente en une teneur allant de 0,1 à 15 % en poids, par rapport au poids total de la composition, de préférence de 0,5 à 10 % en poids.

13. Composition selon l'une quelconque des revendications précédentes, **caractérisée en ce qu'**elle comprend une matière colorante additionnelle différente des terres colorantes.

14. Composition selon l'une quelconque des revendications précédentes, **caractérisée en ce que** la composition se présente sous une forme d'émulsion ou de poudre, libre ou compactée.

15. Procédé de maquillage de la peau ou des lèvres comprenant l'application sur la peau ou les lèvres d'une composition selon l'une quelconque des revendications précédentes.

## Claims

1. Cosmetic composition comprising, in a physiologically acceptable medium, at least one composite particle, otherwise known as colouring earth, which exists in the natural state and which comprises a colouring inorganic part and a noncolouring inorganic part, and at least one mattifying filler.

2. Composition according to the preceding claim, **characterized in that** the colouring earths are composite particles comprising a colouring inorganic part comprising iron, preferably in the oxidized form.

3. Composition according to either one of the preceding claims, **characterized in that** the colouring earths comprise coloured metal oxides other than iron oxide, such as manganese oxide or aluminium oxide.

4. Composition according to any one of the preceding claims, **characterized in that** the colouring earths are chosen from yellow earths, red earths, green earths, brown earths or ombre earths and their mixtures.

5. Composition according to any one of the preceding claims, **characterized in that** the colouring earth comprises a noncolouring inorganic part chosen from clay, gypsum, calcite, magnesium carbonate, quartz, hydrated alumina and their mixtures.

6. Composition according to any one of the preceding claims, **characterized in that** the colouring earths comprise at least one colouring inorganic part and at least one noncolouring inorganic part, the colouring inorganic part being integral with one at least of the constituents of the noncolouring inorganic part.

7. Composition according to any one of the preceding claims, **characterized in that** the mattifying filler has a refractive index of less than or equal to 2.2, in particular of less than or equal to 2, especially of less than or equal to 1.8 and more particularly varying from 1.3 to 1.6.

8. Composition according to any one of the preceding claims, **characterized in that** the mattifying filler has a volume-average particle size of less than 40 µm, preferably of less than 30 µm and more preferably of less than 20 µm.

9. Composition according to any one of the preceding claims, **characterized in that** the mattifying filler confers, on the said composition, a mattifying power of less than 0.75.

10. Composition according to any one of the preceding claims, **characterized in that** the mattifying filler has a specific surface for the particles of greater than 10 m²/g and in particular of greater than 50 m²/g.

11. Composition according to any one of the preceding claims, **characterized in that** the mattifying filler is chosen from:
- porous silica micorparticles,
- polytetrafluoroethylene powders,
- silicone resin powders,
- hollow hemispherical silicone particles,
- acrylic copolymer powders,
- wax powders,
- polyethylene powders,
- crosslinked organopolysiloxane elastomer powders coated with silicone resin,
- composite talc/titanium dioxide/alumina/silica powders,
- polyamide powders, and
- their mixtures.

12. Composition according to any one of the preceding claims, **characterized in that** the mattifying filler is present in a content ranging from 0.1 to 15% by weight, with respect to the total weight of the composition, preferably from 0.5 to 10% by weight.

13. Composition according to any one of the preceding claims, **characterized in that** it comprises an additional colouring material other than the colouring earths.

14. Composition according to any one of the preceding claims, **characterized in that** the composition is provided in an emulsion or loose or compact powder form.

15. Method for making up the skin or lips, comprising the application, to the skin or lips, of a composition according to any one of the preceding claims.

## Patentansprüche

1. Kosmetische Zusammensetzung, die in einem physiologisch akzeptabeln Medium mindestens ein natürlich vorkommendes Verbundpartikel, das auch als Erdfarbe bezeichnet wird und das einen farbigen mineralischen Teil und einen nicht farbigen mineralischen Teil aufweist, und mindestens einen mattierenden Füllstoff enthält.

2. Zusammensetzung nach dem vorhergehenden Anspruch, **dadurch gekennzeichnet, dass** die Erdfarben Verbundpartikel sind, die einen farbigen mineralischen Teil aufweisen, der Eisen vorzugsweise in Oxidform enthält.

3. Zusammensetzung nach einem der vorhergehenden Ansprüche, **dadurch gekennzeichnet, dass** die Erdfarben von Eisenoxid verschiedene farbige Metalloxide enthalten, wie Manganoxid oder Aluminiumoxid.

4. Zusammensetzung nach einem der vorhergehenden Ansprüche, **dadurch gekennzeichnet, dass** die Erdfarben unter den gelben Erdfarben, roten Erdfarben, grünen Erdfarben, braunen Erdfarben, Umbra und deren Gemischen ausgewählt ist.

5. Zusammensetzung nach einem der vorhergehenden Ansprüche, **dadurch gekennzeichnet, dass** die Erdfarbe mindestens einen nicht farbigen mineralischen Teil aufweist, der unter Ton, Gips, Calcit, Magnesiumcarbonat, Quarz, Aluminiumhydroxid und deren Gemischen ausgewählt ist.

6. Zusammensetzung nach einem der vorhergehenden Ansprüche, **dadurch gekennzeichnet, dass** die Erdfarben mindestens einen farbigen mineralischen Teil und mindestens einen nicht farbigen mineralischen Teil aufweisen, wobei der farbige mineralische Teil mit mindestens einem der Bestandteile des nicht farbigen mineralischen Teils fest verbunden ist.

7. Zusammensetzung nach einem der vorhergehenden Ansprüche, **dadurch gekennzeichnet, dass** der mattierende Füllstoff einen Brechungsindex von kleiner oder gleich 2,2, insbesondere von kleiner oder gleich 2, besonders von kleiner oder gleich 1,8 und ganz besonders im Bereich von 1,3 bis 1,6 aufweist.

8. Zusammensetzung nach einem der vorhergehenden Ansprüche, **dadurch gekennzeichnet, dass** der mattierende Füllstoff eine auf das Volumen bezogene mittlere Partikelgröße unter 40 µm, vorzugsweise unter 30 µm und noch bevorzugter unter 20 µm aufweist.

9. Zusammensetzung nach einem der vorhergehenden Ansprüche, **dadurch gekennzeichnet, dass** der mattierende Füllstoff der Zusammensetzung ein Mattierungsvermögen unter 0,75 verleiht.

10. Zusammensetzung nach einem der vorhergehenden Ansprüche, **dadurch gekennzeichnet, dass** der mattierende Füllstoff eine spezifische Partikeloberfläche über 10 m²/g und insbesondere über 50 m²/g aufweist.

11. Zusammensetzung nach einem der vorhergehenden Ansprüche, **dadurch gekennzeichnet, dass** der mattierende Füllstoff ausgewählt ist unter:
• porösen Siliciumoxidmikropartkeln,
• Polytetrafluorethylenpulvern,
• Siliconharzpulvern,
• halbkugelförmigen, hohlen Siliconpartiken,
• Acrylcopolymerpulvern,
• Wachspulvern,
• Polyethylenpulvern,
• Pulvern eines mit Siliconharz umhüllten, vernetzten, elastomeren Organopolysiloxans,
• aus Talk/Titandioxid/Aluminiumoxid/Siliciumoxid zusammengesetzten Pulvern,
• Polyamidpulvern, und
• deren Gemischen.

12. Zusammensetzung nach einem der vorhergehenden Ansprüche, **dadurch gekennzeichnet, dass** der mattierende Füllstoff in einem Mengenanteil im Bereich von 0,1 bis 15 Gew.-%, bezogen auf das Gesamtgewicht der Zusammensetzung, und vorzugsweise im Bereich von 0,5 bis 10 Gew.-% enthalten ist.

13. Zusammensetzung nach einem der vorhergehenden Ansprüche, **dadurch gekennzeichnet, dass** sie ein von den Erdfarben verschiedenes ergänzendes Farbmittel enthält.

14. Zusammensetzung nach einem der vorhergehenden Ansprüche, **dadurch gekennzeichnet, dass** die Zusammensetzung in Form einer Emulsion oder als loses oder kompaktiertes Pulver vorliegt.

15. Verfahren zum Schminken der Haut oder der Lippen, das das Auftragen einer Zusammensetzung nach einem der vorhergehenden Ansprüche auf die Haut oder der Lippen umfasst.
